# EUROPEAN PATENT APPLICATION

(11) **EP 3 653 120 A1**
(43) Date of publication of application: **20.05.2020**
(21) Application number: 18206436.0
(22) Date of filing: 15.11.2018
(51) Int. Cl.: A61B 5/11, A61B 5/00

(54) **A REHABILITATION DEVICE AND A METHOD OF MONITORING HAND POSITIONS**

(71) Applicant: Koninklijke Philips N.V., 5656 AG Eindhoven (NL)
(72) Inventor: JIN, Sheng, 5656 AE Eindhoven (NL); WANG, Jin, 5656 AE Eindhoven (NL); WANG, Xiang, 5656 AE Eindhoven (NL)
(74) Representative: de Haan, Poul Erik

(57) **Abstract**

A rehabilitation device is for monitoring hand positions of a user. It comprises two camera modules which experience different occlusion of finger joints. If finger joints are occluded in the image from one of the camera modules, the location obtained from the image from the other one is used. If finger joints are not occluded in either of the images a location may for example be obtained from the images from both of the camera modules.

## Description

### FIELD OF THE INVENTION

This invention relates to a rehabilitation device, and in particular to a hand rehabilitation device which monitors movement and hand shapes of a user's hand.

### BACKGROUND OF THE INVENTION

Movement therapy is used to rehabilitate patients who have diminished motor control; for example, movement therapy may be suitable for a stroke patient who has reduced control of their hand. Rehabilitation devices are used to facilitate movement therapy. These devices may guide, monitor and measure how patients conduct exercises, give feedback on exercise quality and motivate patients to comply with a training plan.

Some rehabilitation devices use video cameras to track a user's hand movements, whilst other rehabilitation devices use active sensors to detect hand movements. Active sensor rehabilitation devices are, in general, more expensive than video camera rehabilitation devices. Furthermore, an active sensor system typically requires a glove to be worn by the user, which may be uncomfortable as a result of the injury being treated.

For a camera based system, a tracking system may be used which includes a video camera for capturing hand movement in the RGB color field and a depth sensor for obtaining depth information. The video camera records an image of the patient's hand, which is processed to localize the hand in the image and to segment the hand from the background of the image, in order to track the position of the patient's hand and movement of the fingers. Segmentation may be performed based on color, since skin color may be easily distinguished from the background of the image. The patient may wear colored markers to improve the accuracy of detection. Other 3D camera designs are also known.

The hand position is typically derived by detecting the position in 3D space of the finger joints. For a camera based system, occlusion of the joints is a problem for hand tracking, since for certain exercises, one or more fingers or the palm may cover another finger or fingers, which means the camera is unable to capture enough information to recover the whole hand gesture. Normally to solve this problem, the positions of the occluded finger joints are recovered based on the available information from the non-occluded fingers and palm, based on a hand shape model. However, the positions of the fingers is then a prediction, and hence may not be accurate.

There is therefore a need for an improved way of monitoring hand shape within a hand rehabilitation system.

### SUMMARY OF THE INVENTION

The invention is defined by the claims.

According to examples in accordance with an aspect of the invention, there is provided a rehabilitation device for monitoring hand positions of a user, comprising:
a support having a hand support region for receiving a user's hand in an upright position;
a first camera module;
a second camera module mounted at a different location to the first camera module such that it experiences different occlusion of finger joints; and
a controller adapted:
   to identify the locations of the finger joints in the images from each of the first and second camera modules;
   to identify which finger joints, if any, are occluded in the images from each of the first and second camera modules;
   for finger joints which are occluded in the image from one of the first and second camera modules, to use the location obtained from the image from the other one of the first and second camera modules as the final joint location;
   for finger joints which are not occluded in either of the images from the first and second camera modules, to use the location obtained from the image from either one of the first and second camera modules as the final joint location, or to use the locations obtained from the images from both of the first and second camera modules to derive the final joint location; and
   determine the hand position at a given time based on the final joint locations.

This device is able to track the hand position by taking two images, and using only one image for finger joints when these joints are occluded in the other image. The support allows the hand to have free movement while supporting the wrist and hand so that patients with weak muscle strength may use the device. For post-stroke patients, there is typically paralysis on one side, which may be the left or right. The device is able to be used with either hand. Note that the term "hand position" is intended to denote the hand shape i.e. state of bending of the fingers and/or the hand position in space.

The images are used as a minimum to extract finger joint locations. Preferably, fingertip locations are also obtained. It should be understood that the term finger in this context is intended to include the thumb. Thus, the term finger is used generally to denote all of the digits of the hand.

The hand support region is for example transparent, wherein the first camera module is mounted beneath the hand support region facing the hand support region and located to one side of the hand support region and the second camera module is mounted beneath the hand support region facing the hand support region and located to an opposite side of the hand support region.

This defines a design in which the camera modules are in a protected and out of the way location beneath the hand support region. The first and second camera modules may be equally spaced to the sides of a center of the hand support region. The coordinate system of the two cameras may then be aligned easily.

The device may further comprise a housing comprising a cavity in which the first and second camera modules are mounted, wherein the hand support region is mounted over a top of the cavity.

This provides a closed unit which is thus protected from debris and damage.

The controller may be adapted:
for finger joints which are not occluded in either of the images from the first and second camera modules, to use an average of the locations obtained from the images from both of the first and second camera modules as the final joint location.

Thus, when a joint is non-occluded in both images, an average position may be obtained. This is likely to be more accurate than the locations obtained from either of the individual images.

The controller may be adapted to determine hand movement based on the determined hand positions over time.

Thus, gestures may be monitored as well as static hand positions.

The first and second camera modules may each comprise a 3D camera. This avoids the need for the patient to wear a glove. Each camera module may comprise two cameras enabling stereo vision. Infrared lights and an infrared filtering system may be used to segment the hand from the background.

The device may further comprise a glove with markers for identification in the captured images.

This needs the patient to wear a glove, but it allows lower cost camera solutions to be adopted. For example 2D camera modules may be used. For example the markers may have different colors and a 2D coordinate location may be obtained by a single RGB camera.

The controller may be adapted to determine whether the hand is a left hand or a right hand based on identifying the side faced by the palm.

The system is then able to monitor the hand movements required by the rehabilitation exercise for the particular hand which is present on the support.

The device for example comprises a display for providing instructions for a user to perform particular hand rehabilitation exercises and for providing information relating to a success level in performing the hand rehabilitation exercises. This provides an interactive rehabilitation exercise.

The controller may be adapted to:
generate a display output comprising the instructions for a user;
analyze movement of the hand of the user; and
generate an output which indicates the success level in performing the hand rehabilitation exercises.

A user data storage device may be provided, which is readable by the controller to identify which hand is to be rehabilitated and the exercises to be implemented. The device thus adapts automatically to the particular rehabilitation regime of the user.

The invention also provides a method of monitoring the hand position of a user of a rehabilitation system, comprising:
receiving the hand of a user in an upright position on a hand support region of a support;
obtaining a first image of the hand using a first camera module;
obtaining a second image of the hand using a second camera module mounted at a different location to the first camera module such that it experiences different occlusion of finger joints;
identifying the locations of the finger joints in the first and second images;
identifying which finger joints, if any, are occluded in the first and second images;
for finger joints which are occluded in one of the first and second images, using the location obtained from the other of the first and second images as the final joint location;
for finger joints which are not occluded in either of the first and second images, using the location obtained from either one of the first and second images as the final joint location, or using the locations obtained from both the first and second images to derive the final joint location; and
determining the hand position based on the final joint locations.

This is the method implemented by the device described above.

The hand support region is for example transparent, the first image is obtained using a first camera module mounted beneath the support facing the hand support region and located to one side of the hand support region, and the second image is obtained using a second camera module mounted beneath the hand support region facing the hand support region and located to an opposite side of the hand support region. The images are thus obtained from below, through the transparent support.

The method may comprise:
for finger joints which are not occluded in either of the first and second images, using an average of the locations obtained from both of the first and second images as the final joint location. Hand movement may be determined based on the determined hand positions over time. The images are for example captured using 3D cameras and the method may comprise determining whether the hand is a left hand or a right hand based on identifying the side faced by the palm.

The method may comprise providing instructions for a user to perform particular hand rehabilitation exercises and providing information relating to a success level in performing the hand rehabilitation exercises as display outputs. User data may be received from a user data storage device which identifies which hand is to be rehabilitated and the exercises to be implemented.

The invention also provides a rehabilitation method, comprising:
generating a display output comprising instructions for a user to perform particular hand rehabilitation exercises;
monitoring hand position using the method described above;
analyzing hand positions and/or movements; and
generating an output which indicates a success level in performing the hand rehabilitation exercises.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a hand rehabilitation system;
Figure 2 shows one example of the hand support in more detail, in end view;
Figure 3 shows one example of the hand support in more detail, in plan view; and
Figure 4 shows a hand rehabilitation method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a rehabilitation device for monitoring hand positions of a user, comprising two camera modules which experience different occlusion of finger joints. If finger joints are occluded in the image from one of the camera modules, the location obtained from the image from the other one is used. If finger joints are not occluded in either of the images a location may for example be obtained from the images from both of the camera modules.

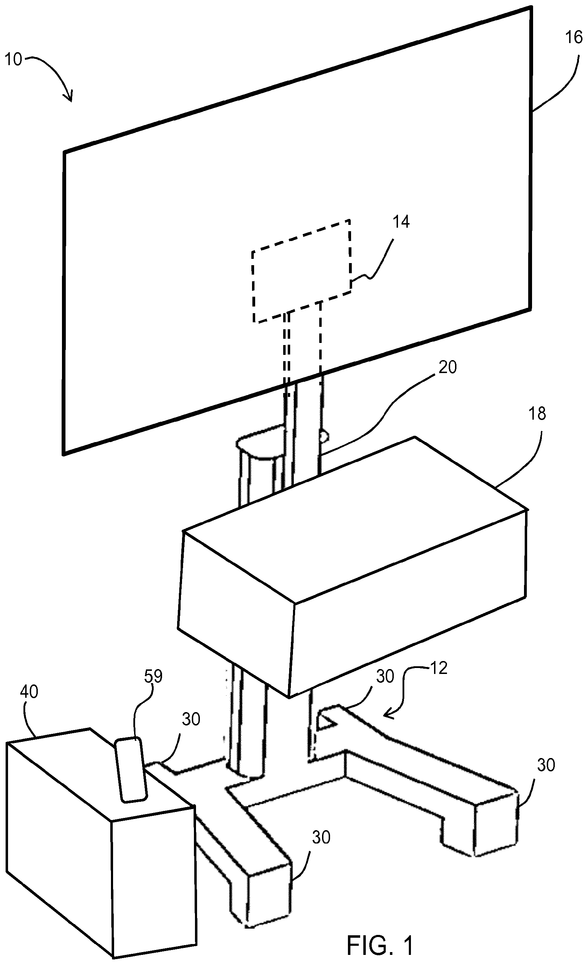

Figure 1 shows a rehabilitation device 10 for monitoring hand movement of a user. It comprises a frame 12 having a display mounting 14 for attachment of a display 16. The display 16 is positioned in front of and above a support 18 also mounted to the frame 12. The support 18 is for imaging the hands of the user as explained further below. The frame 12 has an upright member 20, and the support 18 is mounted to the upright member with adjustable height.

Similarly, the display 16 may optionally also have an adjustable height on the upright member of the frame 12.

The support 18 may be mounted to the upright member 20 with an adjustable elevation angle. This angle may be used to set different types of rehabilitation exercise and difficulty.

In the example shown, the frame 12 is a wheeled trolley having four feet 30 which have rollers at their underside (not shown). The device is then a portable unit which can be wheeled to any location. The user simply brings a chair to sit in front of the device and perform the rehabilitation procedure.

The device comprises a controller 40 which is used to analyze the captured images and display information relating to the rehabilitation. For example, the controller generates a display output comprising instructions for a user to perform particular hand rehabilitation exercises. The movement of a hand or both hands of the user is then analyzed. An output is then provided which indicates a success level in performing the hand rehabilitation exercises.

As is well known, the exercises may be modified in dependence on the performance of the user. The exercise may involve a game play situation to make the experience more entertaining. This invention is not concerned with the rehabilitation procedure itself so further details will not be provided.

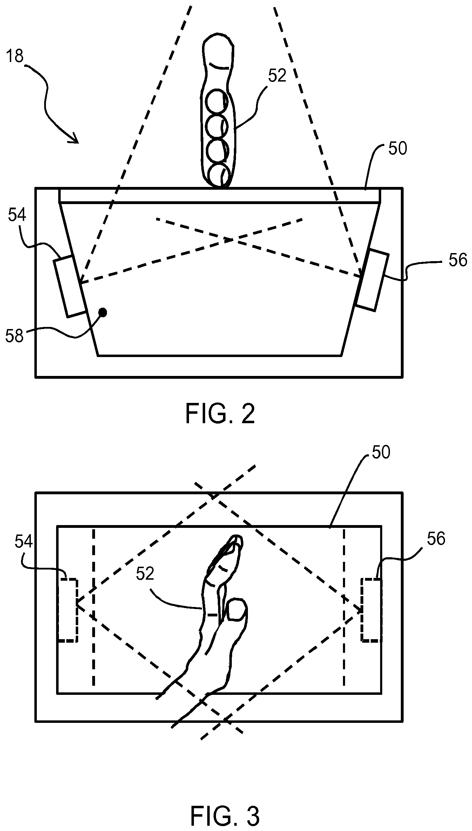

The invention relates to the design of the support 18. An example is shown in Figure 2 from in front and in Figure 3 from above.

The support 18 has a hand support region 50 for receiving a user's hand 52 in an upright position. In the particular example shown, the hand support region 50 is transparent so that an image may be captured through it.

There is a first camera module 54 and a second camera module 56. They are mounted on opposite regions of the hand so that the two camera modules experience different occlusion of finger joints. Thus, together the two camera modules preferably capture non-occluded images of all of the joints and the finger tips.

The captured images are processed by the controller 40, which identifies the locations of the finger joints (and finger tips and optionally other joints in the hand or wrist) in the images from each of the first and second camera modules.

It also identifies which finger joints and finger tips, if any, are occluded in the images from each of the first and second camera modules. It may be that for one camera module, there are fingers, or the palm or knuckles which occlude a particular joint. For finger joints and tips which are occluded in the image from one of the first and second camera modules, the location obtained from the image from the other one of the first and second camera modules is used as the final joint or tip location.

For finger joints and tips which are not occluded in either of the images from the first and second camera modules, one option is to use the location obtained from the image from either one of the first and second camera modules as the final joint and tip location. However, an alternative is to use the locations obtained from the images from both of the first and second cameras to derive the final joint location. This may involve finding an average location. The hand position and shape (i.e. which depends on the bending of the fingers) at a given time is based on set of the final joint locations.

Thus, if there is no occlusion for one camera module, that camera module alone may define the final joint locations. If there is occlusion to both camera modules for different joints, the final locations will be built up from both camera modules. For example, a first set of joints may be determined from one camera module, a second set of joints may be determined from the other cameral module and a last set of joints may be determined from either or both camera modules.

The support is designed such that the instructed gestures should not result in occlusion from both camera modules. However, there may be gestures where there is occlusion from both camera modules. For example, a clenched fist may have the thumb covering some finger joints from all viewing directions. As is known, a hand model may then predict the location of the hidden joint. The predicted location may be based on applying all of the other final joint locations (where there was no occlusion to either camera module or occlusion for only one camera module) to the hand model. Thus, the number of predictions is still reduced.

The vertical hand position means the hand has free movement while the wrist and hand are supported. This assists users with weak muscle strength.

The camera modules may be provided at opposite regions so that occlusions are eliminated by combining the images. These regions may be to opposite sides, or above and below etc. In the example shown, the first and second camera modules 54,56 are mounted beneath the hand support region 50 facing the hand support region. They are located at opposite sides of the hand support region, preferably symmetrically to the sides from the center of the hand support region 50. The coordinate systems of the two cameras are aligned as part of the image processing.

The camera modules are in this way mounted in a cavity 58 which is closed by the hand support region 50. This protects the camera modules and keeps them clean.

The controller 40 interprets the hand positions over time so that both static hand positions and gestures may be identified.

In a first example, each camera module comprises a 3D camera, which in practice combines two cameras to obtain a stereoscopic image, i.e. with depth sensing. There are commercially available suitable depth sensing cameras, such as the Leap Motion (trade mark) camera. The 3D camera for example uses an array of infrared LEDs and has a filtered camera lens. Image processing enables the camera to segment the hand out of the background image and recognize hand gestures. Cameras of this type are able to locate finger joints, finger tips and other hand joints in space without the need to wear a special glove.

In a second example, the user is required to wear a glove with markers for identification in the captured images. This has the disadvantage that it needs the user to wear a glove, but it allows lower cost camera solutions to be adopted. For example 2D camera modules may be used. For example the markers may have different colors and a 2D coordinate location may be obtained by a single RGB camera. By combining two cameras, a 3D location is obtained. If one color maker is occluded by one 2D camera, then it may be visible by another 2D camera. By combining all the information acquired by the two cameras together, the occlusion effect can be reduced.

This second example is thus less preferred in that an occlusion will mean that 3D information is lost so that some extrapolation will be required to reconstruct the full set of 3D locations.

The hand shape recognition allows the controller to determine whether the hand is a left hand or a right hand, for example based on identifying the side faced by the palm. The system is then able to monitor the hand movements required by the rehabilitation exercise for the particular hand which is present on the support. Each user may have a data storage device, which is readable by the controller 40 to identify which hand is to be rehabilitated and the exercises to be implemented.

Figure 1 schematically shows such a data storage device 59 plugged into the controller 40. The overall system thus adapts automatically to the particular rehabilitation regime of the user. It can output an alarm if it is detected that the wrong hand has been presented.

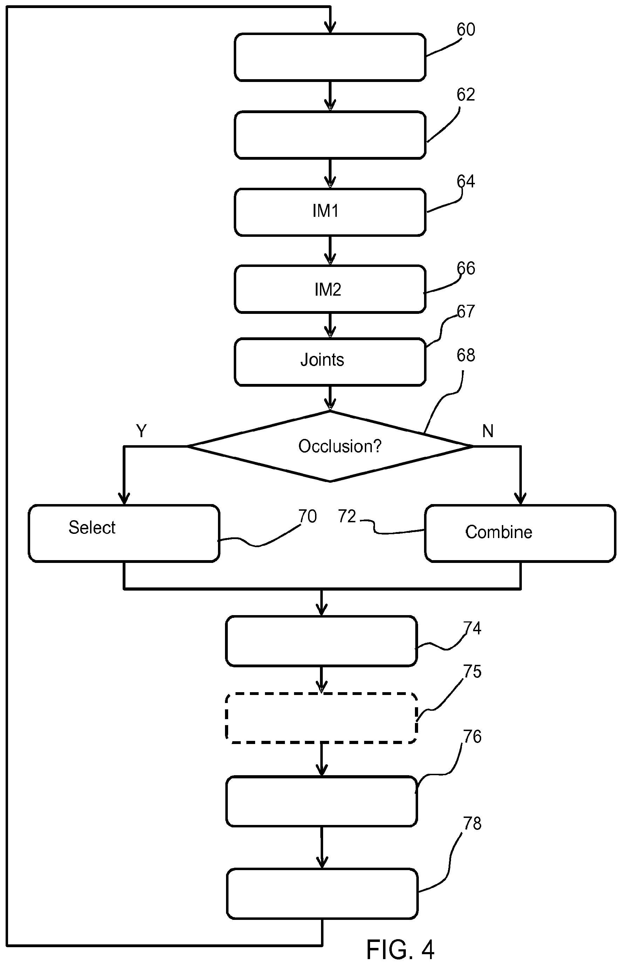

Figure 4 shows a rehabilitation method. In step 60, a display output is generated comprising instructions for a user to perform particular hand rehabilitation exercises. The hand positions are then monitored. This involves receiving the hand of a user in an upright position on a hand support region of a support, in step 62.

In step 64 a first image of the hand is obtained using a first camera module.

In step 66, a second image of the hand is obtained using a second camera module.

In step 67, the locations of the finger joints and tips are determined in the first and second images.

In step 68 it is identified which finger joints and tips, if any, are occluded in the first and second images.

If finger joints and tips are identified which are occluded in one of the first and second images, then in step 70 the location obtained from the other of the first and second images is selected as the final joint or tip location.

If finger joints and tips are identified which are not occluded in either of the first and second images, the location obtained from either one of the first and second images are used as the final joint or tip location, or else the locations obtained from both the first and second images are used to derive the final joint or tip location, such as by taking an average location. This takes place in step 72.

In step 74, the hand position is determined based on the final joint and tip locations.

In step 76, static hand positions and/or hand movements are analyzed.

In step 78, an output is provided which indicates a success level in performing the hand rehabilitation exercises.

The method returns to the step 60 and the instructions will evolve over time to follow a rehabilitation program.

The method may additionally comprises step 75 of determining whether the hand is a left hand or a right hand based on identifying the side faced by the palm. This may take place before any gesture instructions are provided in step 60, so that an initial hand position recognition takes place before any gesture instructions are provided. For example, the first instruction in step 60 may simply be an instruction to place the hand on the hand support region.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single controller or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A rehabilitation device for monitoring hand positions of a user, comprising:
a support (18) having a hand support region (50) for receiving a user's hand in an upright position;
a first camera module (54);
a second camera module (56) mounted at a different location to the first camera module such that it experiences different occlusion of finger joints; and
a controller (40) adapted:
to identify the locations of the finger joints in the images from each of the first and second camera modules;
to identify which finger joints, if any, are occluded in the images from each of the first and second camera modules;
for finger joints which are occluded in the image from one of the first and second camera modules, to use the location obtained from the image from the other one of the first and second camera modules as the final joint location;
for finger joints which are not occluded in either of the images from the first and second camera modules, to use the location obtained from the image from either one of the first and second camera modules as the final joint location, or to use the locations obtained from the images from both of the first and second camera modules to derive the final joint location; and
determine the hand position at a given time based on the final joint locations.

2. A device as claimed in claim 1, wherein the hand support region (50) is transparent, wherein the first camera module (54) is mounted beneath and facing the hand support region and located to one side of the hand support region and the second camera module is mounted beneath and facing the hand support region and located to an opposite side of the hand support region.

3. A rehabilitation device as claimed in claim 2, further comprising a housing comprising a cavity (58) in which the first and second camera modules (54, 56) are mounted, wherein the hand support region (50) is mounted over a top of the cavity.

4. A rehabilitation device as claimed in claim 1, wherein the controller (40) is adapted:
for finger joints which are not occluded in either of the images from the first and second camera modules, to use an average of the locations obtained from the images from both of the first and second camera modules as the final joint location.

5. A rehabilitation device as claimed in claim 1, wherein the controller (40) is adapted to determine hand movement based on the determined hand positions over time.

6. A rehabilitation device as claimed in claim 1, wherein the first and second camera modules (54, 56) each comprise a 3D camera.

7. A rehabilitation device as claimed in claim 1, further comprising a glove with markers for identification in the captured images.

8. A rehabilitation device as claimed in claim 1, wherein the controller (40) is adapted to determine whether the hand is a left hand or a right hand based on identifying the side faced by the palm.

9. A device as claimed in claim 1, comprising a display (16) for providing instructions for a user to perform particular hand rehabilitation exercises and for providing information relating to a success level in performing the hand rehabilitation exercises.

10. A device as claimed in claim 9, wherein the controller (40) is further adapted to:
generate a display output comprising the instructions for a user;
analyze movement of the hand of the user; and
generate an output which indicates the success level in performing the hand rehabilitation exercises.

11. A device as claimed in claim 10, further comprising a user data storage device (59), which is readable by the controller (40) to identify which hand is to be rehabilitated and the exercises to be implemented.

12. A method of monitoring the hand position of a user of a rehabilitation system, comprising:
(62) receiving the hand of a user in an upright position on a hand support region of a support;
(64) obtaining a first image of the hand using a first camera module;
(66) obtaining a second image of the hand using a second camera module mounted at a different location to the first camera module such that it experiences different occlusion of finger joints;
(67) identifying the locations of the finger joints in the first and second images;
(68) identifying which finger joints, if any, are occluded in the first and second images;
for finger joints which are occluded in one of the first and second images, (70) using the location obtained from the other of the first and second images as the final joint location;
for finger joints which are not occluded in either of the first and second images, using (72) the location obtained from either one of the first and second images as the final joint location, or using the locations obtained from both the first and second images to derive the final joint location; and
(74) determining the hand position based on the final joint locations.

13. A method as claimed in claim 12, comprising:
for finger joints which are not occluded in either of the first and second images, using (72) an average of the locations obtained from both of the first and second images as the final joint location.

14. A method as claimed in claim 12, comprising determining hand movement based on the determined hand positions over time.

15. A rehabilitation method, comprising:
(60) generating a display output comprising instructions for a user to perform particular hand rehabilitation exercises;
monitoring hand position using the method of claim 12;
(76) analyzing hand positions and/or hand movements; and
(78) generating an output which indicates a success level in performing the hand rehabilitation exercises.
